# EUROPÄISCHE PATENTSCHRIFT

(11) **EP 0 317 913 B1**
(45) Veröffentlichungstag und Bekanntmachung des Hinweises auf die Patenterteilung: **20.01.1993**
(21) Anmeldenummer: 88119196.9
(22) Anmeldetag: 18.11.1988
(51) Int. Cl.: C07C 69/54, C07C 67/03, C09D 4/00

(54) **Neue Di(meth)acrylate, Verfahren zu deren Herstellung und deren Verwendung**
Di(meth)acrylates, process for their preparation and their use
Di(méth)acrylates, procédé pour leur préparation et leur utilisation

(30) Priorität: 26.11.1987 DE 3740037
(43) Veröffentlichungstag der Anmeldung: 31.05.1989
(73) Patentinhaber: BASF Aktiengesellschaft, 67063 Ludwigshafen (DE)
(72) Erfinder: Merger, Franz, Dr., D-6710 Frankenthal (DE); Liebe, Joerg, Dr., D-6710 Frankenthal (DE); Weiss, Wolfram, Dr., D-6704 Mutterstadt (DE)

(56) Entgegenhaltungen:
- US-A- 4 165 400

## Beschreibung

Die vorliegende Erfindung betrifft neue Di(meth)acrylate, ein Verfahren zu deren Herstellung und deren Verwendung.

Diester aus Diolen und Acrylsäure oder Methacrylsäure, z.B. Tripropylenglykoldiacrylat (TPGDA), 1,6-Hexandioldiacrylat (HDDA) oder Tetraethylenglykoldiacrylat (TEGDA) besitzen als difunktionelle Monomere, z.B. als Reaktivverdünner bei strahlungshärtenden Systemen, eine große technische Bedeutung. Es besteht jedoch ein Bedarf an neuen Verbindungen dieses Typs, um die Eigenschaften der aus diesem System hergestellten Beschichtungen in Bezug auf Härte bei gleichzeitig guter Haftung und Elastizität zu verbessern.

Der Erfindung lag daher die Aufgabe zugrunde, neue Di(meth)acrylate zur Verfügung zu stellen, die als Reaktivverdünner bei strahlungshärtbaren Systemen eingesetzt werden können, mit denen harte und gleichzeitig elastische Oberflächenbeschichtungen mit guter Haftung hergestellt werden können.

Diese Aufgabe konnte dadurch gelöst werden, daß Di(meth)acrylate der folgenden Formel (I) gefunden wurden: 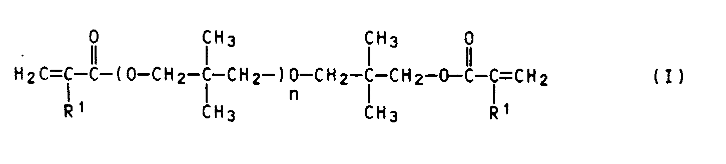
worin
- R¹: Wasserstoff oder Methyl und
- n: die Zahl 1 oder 2 bedeutet.

Verbindungen der Formel (I) sind:
1,7-Bis(acryloyloxi)-2,2,6,6-tetramethyl-4-oxaheptan (Dineopentylglykoldiacrylat),
1,7-Bis(methacryloyloxi)-2,2,6,6-tetramethyl-4-oxaheptan (Dineopentylglykoldimethacrylat),
1,11-Bis(acryloyloxi)-2,2,6,6,10,10-hexamethyl-4,8-dioxaundecan (Trineopentylglykoldiacrylat) und
1,11-Bis(methacryloyloxi)-2,2,6,6,10,10-hexamethyl-4,8-dioxaundecan (Trineopentylglykoldimethacrylat).

Die erfindungsgemäßen Verbindungen der Formel (I) können z.B. dadurch erhalten werden, daß man Diole der Formel (II) 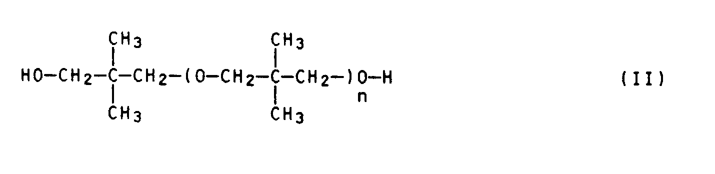
worin
- n: die Zahl 1 oder 2 bedeutet,
mit Acrylsäureestern der allgemeinen Formel (IV) 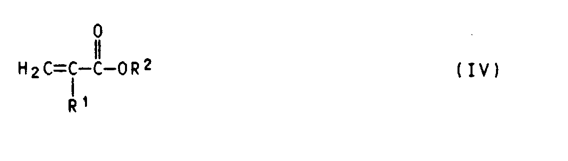
worin
- R¹: Wasserstoff oder Methyl und
- R²: niedere Alkyle mit 1 bis 10 Kohlenstoffatomen bedeuten,
in Gegenwart von Umesterungskatalysatoren umsetzt.

Die Herstellung der Verbindungen der Formel (I) kann aber auch nach anderen, an sich bekannten Methoden erfolgen, z.B. durch Veresterung der Diole der Formel (II) mit Acrylsäure oder Methacrylsäure in Gegenwart von Säuren, oder durch Umsetzung der Diole der Formel (II) mit (Meth)acrylsäurehalogeniden oder (Meth)acrylsäureanhydrid.

Nach einer bevorzugten Ausführungsform des erfindungsgemäßen Verfahrens werden die Diole der Formel (II) mit Acrylsäurealkylestern der Formel (IV) in Gegenwart von Katalysatoren umgesetzt.

Für das erfindungsgemäße Verfahren können Acryl- oder Methacrylsäureester der Formel (IV) verwendet werden, in denen R² einen Alkylrest mit 1 bis 10, vorzugsweise 1 bis 4 Kohlenstoffatomen bedeutet. Beispielsweise seien Butyl-, Propyl-, Ethyl- und insbesondere Methylacrylat oder -methacrylat genannt.

Geeignete Diole (II) sind 2,2,6,6-Tetramethyl-4-oxaheptandiol-1,7 (Dineopentylglykol, DiNPG) und 2,2,6,6,10,10-Hexamethyl-4,8-dioxaundecandiol-1,11 (Trineopentylglykol, TriNPG).

Bei Verwendung von Methylmethacrylat und Dineopentylglykol läßt sich die Umsetzung durch folgende Reaktionsgleichung wiedergeben: 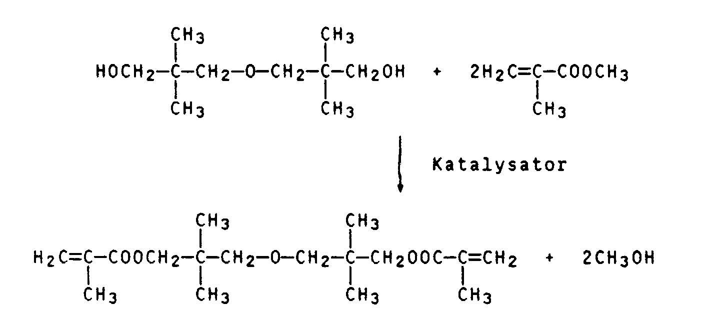
Als Katalysatoren für die Umesterung eignen sich Titanalkoholate oder Chelate der Metalle Titan, Zirkonium, Eisen oder Zink mit 1,3-Dicarbonylverbindungen. Besonders geeignete Titanalkoholate sind Tetramethyl-, Tetrapropyl-, Tetraisopropyl- oder Tetra-n-butyltitanat. Aus dem Bereich der Chelat-Umesterungskatalysatoren eignen sich besonders Metallchelate von 1,4-Diketonen, insbesondere Acetylacetonate, z.B. Titanacetylacetonat oder Zirkoniumacetylacetonat. Die Katalysatoren werden im allgemeinen einzeln oder im Gemisch in Mengen von 0,01 bis 10 Mol.%, bezogen auf die Diole (II) verwendet. Im Fall der Acetylacetonate sind 0,05 bis 1 Mol.%, im Fall der Tetra-C₂-C₄-alkyltitanate 0,2 bis 5 Mol.% vorteilhaft.

Die Ester (IV) können in Mengen von 2 bis 20, vorzugsweise 5 bis 15 Mol pro Mol Diol (II) eingesetzt werden. Die Umsetzung wird zweckmäßigerweise in Gegenwart von üblichen Polymerisationsinhibitoren, z.B. Phenothiazin oder Hydrochinomonomethylether, vorteilhaft in Gegenwart von Sauerstoff durchgeführt. Der Sauerstoff wird in der Regel in Form von Luft in Mengen von 0,1 bis 1 l pro Stunde und Mol Diol (II) angewandt.

Die Umsetzung kann unter Normaldruck, Unter- oder Überdruck durchgeführt werden. Geeignete Reaktionstemperaturen sind 30 bis 150, bevorzugt 50 bis 130, insbesondere 70 bis 120°C. Die Umsetzung kann diskontinuierlich oder kontinuierlich erfolgen. Zweckmäßigerweise kann man so verfahren, daß man die Ausgangsstoffe (II) und (IV) gemeinsam zum Sieden erhitzt und dabei kontinuierlich das freiwerdende Alkanol, gegebenenfalls in Form seines Azeotrops mit dem Ester (II), abdestilliert. Die Reaktionszeiten liegen je nach Reaktionstemperatur und Katalysator bei ca. 1 bis 6 Stunden. Es ist auch möglich, die Umsetzung in Gegenwart eines inerten Lösungsmittels, z.B. Toluol oder Cyclohexan, durchzuführen.

Nach Beendigung der Reaktion kann der Katalysator in an sich bekannter Weise abgetrennt werden. Tetraalkyltitanate lassen sich z.B. nach Hydrolyse mit Wasser durch Filtrieren oder Zentrifugieren abtrennen. In der bevorzugten Ausführungsform wird der Katalysator im Reaktionsgemisch belassen; nach Abdampfen des überschüssigen Esters (IV) wird dann das Produkt in einem Dünnfilmverdampfer destilliert und dadurch vom Katalysator abgetrennt.

Setzt man die erfindungsgemäßen Verbindungen der allgemeinen Strukturformel (I) in z.B. Polyesteracrylat-haltigen Lacken als Reaktivverdünner ein und vergleicht mit den dem Stand der Technik entsprechenden Monomeren Hexandioldiacrylat (HDDA) und Tripropylenglykoldiacrylat (TPGDA), so findet man überraschenderweise eine vorteilhafte Härte-Elastizitäts-Kombination bei der strahlungsgehärteten Lackierung, was sich in hoher Pendeldämpfung DIN 53 157 bei gleichzeitig hoher Erichsentiefung (DIN 53 156) zeigt.

### A) Herstellungsbeispiele

### Beispiel 1

190 g Dineopentylglykol wurden zusammen mit 860 g Acrylsäuremethylester, 3,4 g Tetra-n-butyltitanat und 0,3 g Hydrochinomonomethylether unter Rühren und unter Einleiten von stündlich 0,5 l Luft zum Sieden erhitzt. Über eine Füllkörperkolonne wurde das freigesetzte Methanol als Azeotrop mit Acrylsäuremethylester beim Siedepunkt 62,5°C abdestilliert.

Nach Beendigung der Umsetzung wurde der überschüssige Acrylsäuremethylester bei 20 mbar destilliert und der Rückstand bei 140°C/0,4 mbar mit einem Sambay-Verdampfer destilliert. Man erhielt 289 g Dineopentylglykol-diacrylat (Reinheit >99 %), entsprechend einer Ausbeute von 97 % der Theorie.

### Beispiel 2

Analog dem in Beispiel 1 beschriebenen Verfahren wurden 190 g Dineopentylglykol mit 730 g Methacrylsäuremethylester in Gegenwart von 3,4 g Tetra-n-butyltitanat und 0,3 g Hydrochinonmethylether umgesetzt. Man erhielt nach Aufarbeitung 315 g Dineopentylglykoldimethacrylat vom Siedepunkt 155°C/0,3 mbar, entsprechend einer Ausbeute von 96,5 % der Theorie.

### Beispiel 3

Analog dem in Beispiel 1 beschriebenen Verfahren wurden 190 g Dineopentylglykol mit 1000 g Methacrylsäuremethylester in Gegenwart von 4,8 g Zirkoniumacetylacetonat und 0,3 g Hydrochinonmonomethylether umgesetzt. Man erhielt nach Aufarbeitung 309 g Dineopentylglykoldimethacrylat, entsprechend einer Ausbeute von 95 % der Theorie.

### Beispiel 4

Analog dem im Beispiel 1 beschriebenen Verfahren wurden 276 g Trineopentylglykol mit 1135 g Acrylsäuremethylester in Gegenwart von 6,8 g Tetra-n-butyltitanat und 0,3 g Hydrochinonmonomethylether umgesetzt. Nach Aufarbeitung erhielt man 357 g Trineopentylglykoldiacrylat vom Siedepunkt 170 bis 175°C/0,3 mbar, entsprechend einer Ausbeute von 93 % der Theorie.

### Beispiel 5

Analog dem im Bespiel 1 beschriebenen Verfahren wurden 276 g Trineopentylglykol mit 1000 g Methacrylsäuremethylester in Gegenwart von 6,8 g Tetra-n-butyltitanat und 0,3 g Hydrochinonmonomethylether umgesetzt. Nach Aufarbeitung erhielt man 325 g Trineopentylglykoldimethacrylat vom Siedepunkt 175 bis 180°C/0,4 mbar, entsprechend einer Ausbeute von 79 % der Theorie.

### B) Anwendungsbeispiele

51 g eines Polyesteracrylates (aus 1 Mol Adipinsäure, 2 Mol Ethylenglykol, 0,5 Mol Phthalsäure, 1 Mol Trimethylolpropan und 2,5 Mol Acrylsäure) mit einer Viskosität von 40 Pa·s (23°C) wurden durch Zusatz von 49 g Dineopentylglykoldiacrylat (DiNPGDA) aus Beispiel 1 auf 0,5 Pa·s (23°C) verdünnt. Die Mischung wurde mit 1 g Benzyldimethylketal, 2 g Benzophenon und 3 g Methyldiethanolamin initiiert.

Der so erhaltene Klarlack wurde mit einer Schicht von 50 µm auf phosphatierte Tiefziehbleche aufgezogen und mit einer Quecksilber-Hochdrucklampe (Leistung 120 Watt/cm Bogenlänge) bestrahlt. Die Belichtungsdauer bis zum Erreichen einer kratzfesten Beschichtung betrug 0,4 sec, die Lackierungen wurden 1,8 sec belichtet.

Zur Beurteilung der Lackierungsgüte wurden die Pendeldämpfung nach König (DIN 53 157) und die Erichsentiefung (DIN 53 156) herangezogen. Die Ergebnisse sind Tabelle 1 zu entnehmen.

### Vergleichsbeispiele 1 und 2

Analog zum obigen Beispiel wurde das Polyesteracrylatharz mit 1,6-Hexandioldiacrylat (HDDA) bzw. Tripropylenglykoldiacrylat (TPGDA) verdünnt und initiiert. In Tabelle 1 sind die Bewertungsergebnisse im Vergleich zu Beispiel 6 aufgetragen.

**Tabelle 1**

| | Beispiel 6 | Vergl. 1 | Vergl. 2 |
|---|---|---|---|
| - Verdünner | DiNPGDA | HDDA | TPGDA |
| - Verdünnermenge auf 100 g Klarlack o. Initiator (Visk. = 0,5 Pa·s) | 49 g | 39 g | 49 g |
| - Belichtungszeit bis Kratzfestigkeit | 0,4 sec | 0,3 sec | 0,3 sec |
| - Pendeldämpfung (sec) (DIN 53 157) | 67 | 49 | 39 |
| - Erichsentiefung (mm) (DIN 53 156) | 7,4 | 5,4 | 6,4 |

Aus den 67 sec Pendeldämpfung ist die hohe Härte und aus den 7,4 mm Erichsentiefung ist die hohe Dehnbarkeit bei gleichzeitig guter Haftung der Beschichtung des Beispiels 6 zu erkennen.

## Patentansprüche

1. Di(meth)acrylate der Formel (I) 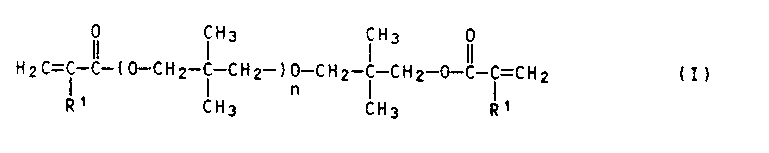 worin
R¹ Wasserstoff oder Methyl und
n die Zahl 1 oder 2 bedeuten.

2. Verfahren zur Herstellung der Di(meth)acrylate gemäß Anspruch 1, dadurch gekennzeichnet, daß man Diole der Formel (II) 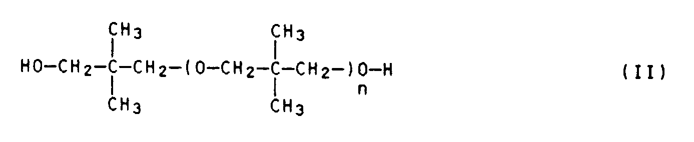 worin
n die Zahl 1 oder 2 bedeutet,
mit (Meth)acrylsäure oder deren Derivaten der allgemeinen Formel (III) 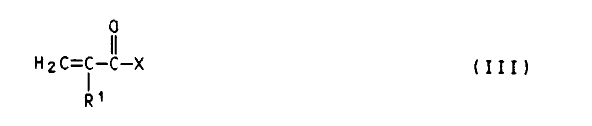 worin
R¹ Wasserstoff oder Methyl bedeuten, und
X für -OH, -OR², -Halogen oder 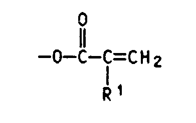
steht mit R² C₁-C₁₀-Alkyl unter allgemein üblichen Veresterungsbedingungen umsetzt.

3. Verfahren zur Herstellung der Di(meth)acrylate nach Anspruch 2, dadurch gekennzeichnet, daß man die Diole der Formel (II) mit den Methacrylsäureestern der Formel (IV) 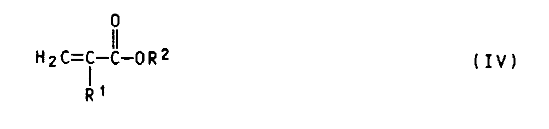 worin
R¹ Wasserstoff oder Methyl bedeuten, und
R² C₁-C₁₀-Alkylgruppen
in Gegenwart von Titanalkoholaten oder Chelatverbindungen aus den Metallen Titan, Zirkonium, Eisen oder Zink mit 1,3-Dicarbonylverbindungen unter Umesterungsbedingungen umsetzt.

4. Verfahren zur Herstellung der Di(meth)acrylate nach Anspruch 2 oder 3, dadurch gekennzeichnet, daß man 2,2,6,6-Tetramethyl-4-oxaheptandiol-1,7 als Diol der Formel (II) einsetzt.

5. Verfahren zur Herstellung der Di(meth)acrylate nach einem der Ansprüche 2 bis 4, dadurch gekennzeichnet, daß man 2,2,6,6,10,10-Hexamethyl-4,8-dioxa-undecandiol-1,11 als Diol der Formel (II) einsetzt.

6. Verwendung der Di(meth)acrylate gemäß Anspruch 1 als Reaktivverdünner in strahlungshärtbaren Lacksystemen.

## Claims

1. A di(meth)acrylate of the formula (I) 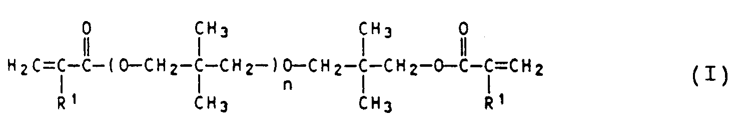 where R¹ is hydrogen or methyl and n is 1 or 2.

2. A process for the preparation of a di(meth)acrylate as claimed in claim 1, wherein a diol of the formula (II) 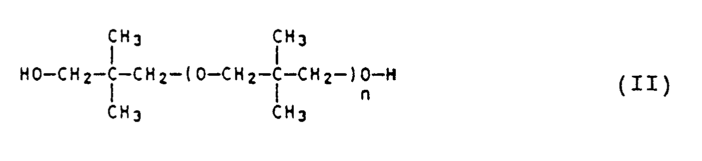 where n is 1 or 2,
is reacted with (meth)acrylic acid or one of its derivatives of the formula (III) 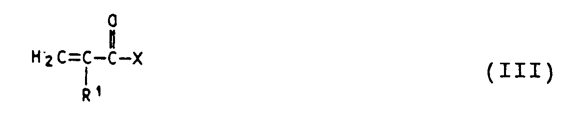 where R¹ is hydrogen or methyl and X is -OH, -OR², -halogen or 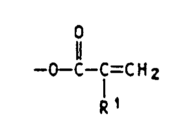 where R² is C₁-C₁₀-alkyl, under generally conventional esterification conditions.

3. A process for the preparation of a di(meth)acrylate as claimed in claim 2, wherein a diol of the formula (II) is reacted with a methacrylate of the formula (IV) 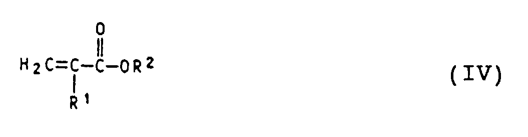 where R¹ is hydrogen or methyl and R² is C₁-C₁₀-alkyl, in the presence of a titanium alcoholate or a chelate compound of the metals titanium, zirconium, iron or zinc with a 1,3-dicarbonyl compound under transesterification conditions.

4. A process for the preparation of a di(meth)acrylate as claimed in claim 2 or 3, wherein a 2,2,6,6-tetramethyl-4-oxaheptane-1,7-diol is used as the diol of the formula (II).

5. A process for the preparation of a di(meth)acrylate as claimed in any of claims 2 to 4, wherein 2,2,6,6,10,10-hexamethyl-4,8-dioxaundecane-1,11-diol is used as the diol of the formula (II).

6. Use of a di(meth)acrylate as claimed in claim 1 as a reactive diluent in radiation-curable coating systems.

## Revendications

1. Di(méth)acrylates de la formule (I) 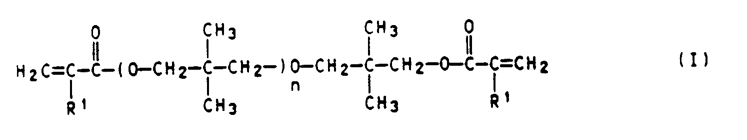 dans laquelle R¹ représente un atome d'hydrogène ou le radical méthyle et
n est égal à 1 ou à 2.

2. Procédé de préparation de di(méth)acrylates suivant la revendication 1, caractérisé en ce que l'on fait réagir des diols de la formule (II) 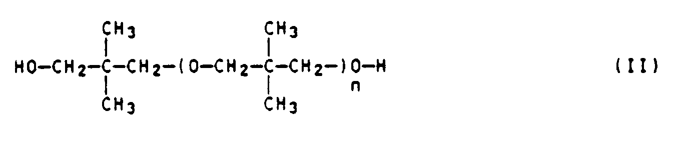 dans laquelle
n est égal à 1 ou à 2,
avec l'acide méthacrylique ou l'acide acrylique ou leurs dérivés de la formule générale (III) 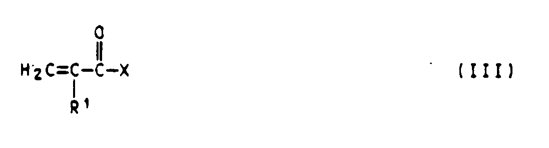 dans laquelle
R¹ représente un atome d'hydrogène ou le radical méthyle et
X représente -OH, -OR², un halogène ou le radical de la formule 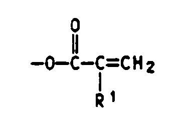 dans laquelle R² représente un radical alkyle en C₁-C₁₀, dans des conditions d'estérification usuelles dans leur ensemble.

3. Procédé de préparation de di(méth)acrylates selon la revendication 2, caractérisé en ce que l'on fait réagir des diols de la formule (II) avec des esters de l'acide méthacrylique de la formule (IV) 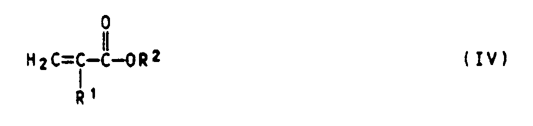 dans laquelle
R¹ représente un atome d'hydrogène ou le radical méthyle et
R² représente des radicaux alkyle en C₁-C₁₀, en présence d'alcoolates de titane ou de composés du type chélate obtenus à partir des métaux titane, zirconium, fer ou zinc avec des composés 1,3-dicarbonylés, dans des conditions de transestérification.

4. Procédé de préparation de di(méth)acrylates selon la revendication ou la revendication 3, caractérisé en ce que l'on utilise le 2,2,6,6-tétraméthyl-4-octaheptanediol-1,7 à titre de diol de la formule (II).

5. Procédé de préparation des di(méth)acrylates selon l'une quelconque des revendications 2 à 4, caractérisé en ce que l'on utilise le 2,2,6,6,10,10-hexaméthyl-4,8-dioxa-undécanediol-1,11 à titre de diol de la formule (II).

6. Utilisation des di(méth)acrylates selon la revendication 1 à titre de diluants réactifs dans des systèmes de peinture ou de laque durcissables par irradiation.
